(19) Europäisches Patentamt
European Patent Office
Office européen des brevets

(11) Veröffentlichungsnummer: **0 060 224**
**A1**

(12) **EUROPÄISCHE PATENTANMELDUNG**

(21) Anmeldenummer: 82810101.4

(22) Anmeldetag: 05.03.82

(51) Int. Cl.[3]: C 23 F 11/12
C 23 F 11/14, C 09 K 5/00
C 07 C 55/02, C 10 M 3/18

(30) Priorität: 09.03.81 GB 8107300

(43) Veröffentlichungstag der Anmeldung: 15.09.82 Patentblatt 82/37

(84) Benannte Vertragsstaaten: DE FR GB IT NL

(71) Anmelder: CIBA-GEIGY AG
Patentabteilung Postfach
CH-4002 Basel(CH)

(72) Erfinder: Howell, Frederick Harold, Dr.
27 High Bank
Atherton Lancashire M29 9HZ(GB)

(54) **Korrosionsschutzmittelzusammensetzung.**

(57) Eine Korrosionsschutzmittelzusammensetzung zur Hemmung der Korrosion von Metallen, gekennzeichnet durch
(a) mindestens eine 3-Alkyladipinsäure der Formel

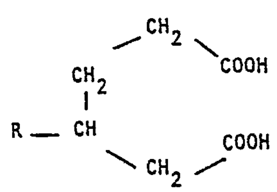

worin R eine geradkettige oder verzweigte $C_4$-$C_9$-Alkylgruppe bedeutet,
(b) ein Basifizierungsmittel und gegebenenfalls
(c) Wasser,
wobei das Gewichtsverhältnis der Verbindung der Formel I zum Basifizierrungsmittel im Bereich 1:1 bis 1:20 liegt, sowie eine Funktionsflüssigkeitsformulierung, die als Korrosionsschutzmittel eine Korrosionsschutzmittelzusammensetzung nach Anspruch 1 enthält, und neue Verbindungen der Formel I.

./...

EP 0 060 224 A1

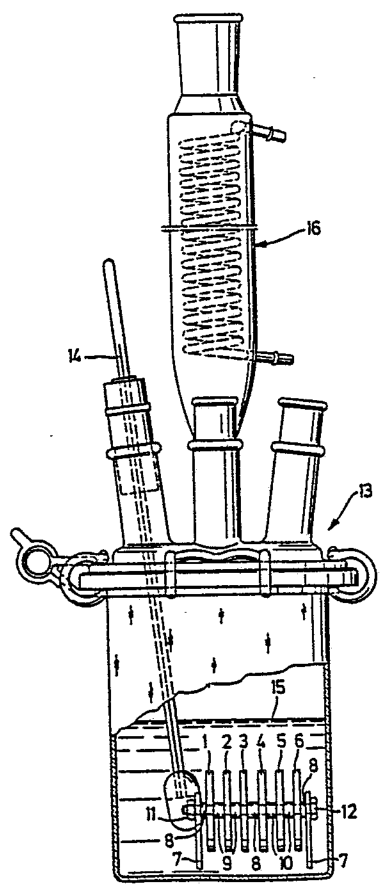

16
14
13
15
1 2 3 4 5 6
8
11
12
8
7
9 8 10
7

# Korrosionsschutzmittelzusammensetzung

Vorliegende Erfindung betrifft neue Korrosionsschutzmittelzusammensetzungen sowie deren Verwendung beim Korrosionsschutz von Metallen.

In der U.S. Patentschrift Nr. 3 696 048 wird eine Korrosionsschutzmittelzusammensetzung beschrieben, welche aus (a) 50-98 Gew.-% eines Salzes einer Dicarbonsäure mit 10-50 Kohlenstoffatomen und eines Kohlenwasserstoffamins mit 10-30 Kohlenstoffatomen und (b) 2-50 Gew.-% einer mindestens eine Hydroxylgruppe enthaltenden solvatisierenden Verbindung, vorzugsweise einem oxyalkylierten, 5-15 Oxyalkylgruppen mit 1 bis 6 Kohlenstoffatomen enthaltenden Alkylphenol, wobei dessen Alkylgruppe 5 bis 15 Kohlenstoffatome enthält, besteht. Als Beispiele für Dicarbonsäuren sind unter anderem Alkyldicarbonsäuren, z.B. Alkyladipinsäure, in der die Alkylgruppe eine langkettige Gruppe ist und mindestens 10 Kohlenstoffatome enthält, angegeben.

Ferner ist aus U.S. Patent 3 907 578 eine Zusammensetzung zum Schutz von Metallen gegen Korrosion durch Berührung mit eine Chloridverunreinigung enthaltendem Wasser bekannt, wobei diese Zusammensetzung aus einem Gemisch aus (a) einem Salz einer Dicarbonsäure mit 10 bis 50 Kohlenstoffatomen und eines aliphatischen Amins mit 10-30 Kohlenstoffatomen, wobei dieses Salz im allgemeinen ein Säure/Aminverhältnis von 1:1 bis 3:1 Aequivalenten Säure pro Aequivalent Amin aufweist, und (b) einem cyclischen Amin oder einem Gemisch aus cyclischen Aminen mit 10 oder weniger Kohlenstoffatomen besteht. Die Dicarbonsäurekomponente kann dabei eine Alkyladipinsäure sein, in der wiederum der Alkylteil langkettig ist und mindestens 10 Kohlenstoffatome enthält.

Weitere bekannte Metallkorrosionsschutzkombinationen aus Triäthanolamin mit unsubstituierter Adipinsäure finden sich z.B. in "The Development of Grinding Fluids of Non-Nitrite Type [Die Entwicklung von Schleifflüssigkeiten vom nitrit-freien Typ]", Hideki Yasui u.a., Kagaku to Kogyo 54, (3), 93-99, 1980.

Gemische aus Natriumnitrit und Triäthanolamin sind ebenfalls zum Korrosionsschutz bei der Metallbearbeitung eingesetzt worden. Wegen der damit verbundenen Toxizitätsprobleme und gesetzlichen Vorschriften über die Abwasserzusammensetzung in manchen Ländern geht jedoch die Industrie von der Verwendung von Natriumnitrit ab.

Zudem besitzen diese bekannten Zusammensetzungen den Nachteil, dass in Systemen, wo das Metall mit Wasser in Berührung steht, die Korrosionsschutzwirkung der von Systemen auf der Grundlage von N-Carboxyalkyl-sulfonamiden unterlegen ist.

Aufgabe der vorliegenden Erfindung ist es, eine Korrosionsschutzmittelzusammensetzung zu schaffen, welche die Nachteile vorbekannter Zusammensetzungen vermeidet und verstärkte Korrosionsschutzwirkung aufweist.

Es wurde nun überraschend gefunden, dass eine Kombination aus gewissen spezifischen Alkyladipinsäuren mit verhältnismässig kurzen Alkylketten und Triäthanolamin diese Aufgabe völlig löst.

Gegenstand vorliegender Erfindung ist dementsprechend eine Korrosionsschutzmittelzusammensetzung zur Verwendung beim Korrosionsschutz von Metallen, insbesondere Eisenmetallen, welche durch (a) mindestens eine 3-Alkyladipinsäure der Formel

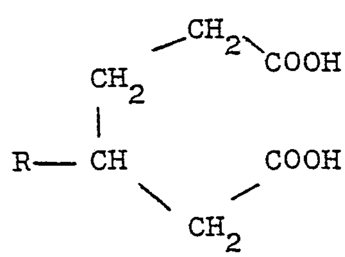

I

worin R eine geradkettige oder verzweigte $C_4$-$C_9$-, vorzugsweise $C_8$-$C_9$-Alkylgruppe bedeutet, b) ein Basifizierungsmittel und c) gegebenenfalls Wasser gekennzeichnet ist, wobei das Gewichtsverhältnis der Verbindung der Formel I zum Basifizierungsmittel im Bereich 1:1 bis 1:20 liegt.

Das Basifizierungsmittel kann z.B. eine wässrige Lösung eines Alkali- oder Erdalkalimetallsalzes, des Ammoniaks oder eines gegebenenfalls substituierten organischen Amins sein. Wässrige Lösungen von Alkalimetallhydroxyden, insbesondere Natrium- oder Kaliumhydroxyd, wässriges Ammoniumhydroxyd und Triäthanolamin werden dabei bevorzugt.

Das dabei verwendete Triäthanolamin kann hochrein oder von technischer Qualität mit praktisch ungefähr 85 Gew.-% Triäthanolamin sein.

Beispiele für Verbindungen der Formel I sind unter anderem: 3-n-Butyladipinsäure, 3-t-Butyladipinsäure, 3-n-Pentyladipinsäure, 3-(1,1-Dimethylpropyl)-adipinsäure, 3-n-Hexyladipinsäure, 3-n-Heptyladipinsäure, 3-n-Octyladipinsäure, 3-(1,1,3,3-Tetramethyl-butyl)-adipinsäure und 3-Nonyladipinsäure.

Gewünschtenfalls kann man zunächst ein Salz einer Verbindung der Formel I durch Umsetzung der Komponenten a) und b) herstellen und dieses Salz dann direkt, gegebenenfalls als Lösung in Wasser, als erfindungsgemässe Korrosionsschutzmittelzusammensetzung einsetzen.

Unter den Verbindugen der Formel I sind die folgenden bekannt: 3-Butyladipinsäure und 3-Hexyladipinsäure (G. Weitzel, H. Queckenstedt, W. Grellmann und H. Lautner, Z. physiol. Chem. 285, 58-77 (1950)), 3-t-Butyladipinsäure und 3-t-Amyladipinsäure (H. Pines und V.N. Ipatieff, JACS, 61, 2728-2730 (1939)) sowie 3-n-Hexyladipinsäure und 3-t-Octyladipinsäure (D.W. Goheen und W.R. Vaughan, J. Org. Chem. 23, 891 (1958)).

Die übrigen Verbindungen sind neu und nach an sich bekannten und in den Beispielen der vorliegenden Anmeldung erläuterten Methoden herstellbar.

Gegenstand vorliegender Erfindung sind demgemäss auch Verbindungen der Formel

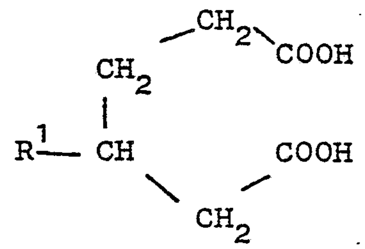

II

worin R' geradkettiges oder verzweigtes $C_7$-$C_9$-Alkyl bedeutet, jedoch mit Ausnahme von t-Octyl.

Die erfindungsgemässe Korrosionsschutzmittelzusammensetzung ist von besonderem Interesse zur Verwendung in wässrigen Metallbearbeitungsformulierungen, z.B. wasserverdünnbaren Schneid- oder Schleifflüssigkeiten, Frostschutzzusammensetzungen und Hydraulikflüssigkeitszusammensetzungen auf Wassergrundlage.

Gegenstand vorliegender Erfindung ist demnach ferner eine Funktionsflüssigkeitsformulierung, welche dadurch gekennzeichnet ist, dass sie als Korrosionsschutzmittel eine wie oben definerte, erfindungsgemässe Korrosionsschutzmittelzusammensetzung enthält.

Unter dem Begriff "wässrige Metallbearbeitungsformulierung" versteht man dabei eine Formulierung, wie sie bei der spanabgebenden Bearbeitung von Metallen, z.B. beim Bohren, Fräsen, Nachbohren, Drehen, Schneiden, Sägen, Schleifen und Gewindeschneiden sowie beim spanlosen Verformen, Ziehen und Walzen verwendet wird.

Die in der Funktionsflüssigkeitsformulierung bei der Verwendung vorliegende Menge der Verbindung der Formel I (oder deren Salz) beträgt zweckmässig 0,005 - 2 Gew.-%, vorzugsweise 0,01 bis 1 Gew.-% und insbesondere 0,05 bis 1 Gew.-%, bezogen auf das Gesamtgewicht der Formulierung.

Metallbearbeitungsflüssigkeiten, in welche die erfindungsgemässe Korrosionsschutzmittelzusammensetzung eingearbeitet werden kann, sind beispielsweise unter anderem:

a) Wässrige Konzentrate des erfindungsgemässen Korrosionsschutzmittels und gegebenenfalls eines oder mehrerer, in

Verdünnungen von 1:50 bis 1:000 verwendeter Verschleisszusätze, wie sie üblicherweise als Schleifflüssigkeiten eingesetzt werden;
b) Polyglykole, die Biozide, das erfindungsgemässe Korrosionsschutzmittel und in Verdünnungen von 1:20 bis 1:40 für Schneidvorgänge sowie 1:60 bis 1:80 zum Schleifen verwendete Verschleisszusätze enthalten;
c) halbsynthetische Schneidflüssigkeiten ähnlich b), die jedoch zusätzlich 10 bis 25 % Oel mit so viel Emulgator enthalten, dass das mit Wasser verdünnte Produkt durchsichtig wird;
d) ein emulgierbares Mineralölkonzentrat, das beispielsweise Emulgatoren, das erfindungsgemässe Korrosionsschutzmittel, Höchstdruck/Verschleisszusätze, Biozide, Entschäumer, Kupplungsmittel usw. enthält; diese werden gewöhnlich 1:10 bis 1:50 mit Wasser zu einer weissen undurchsichtigen Emulsion verdünnt; und
e) ein Produkt ähnlich (d), das weniger Oel und mehr Emulgator enthält und bei Verdünnung auf den Bereich 1:50 bis 1:100 eine durchsichtige Emulsion für Schneid- oder Schleifvorgänge liefert.

Frostschutz- und Hydraulikflüssigkeitsformulierungen auf Wassergrundlage für Kraftfahrzeugmotoren basieren auf wässrigen Glykolen. Solche wässrige Glykollösungen verursachen jedoch Korrosion der damit in Berührung stehenden Metalle. Die erfindungsgemässen Korrosionsschutzmittelzusammensetzungen, gegebenenfalls im Gemisch mit bekannten Korrosionsschutzmitteln, sind äusserst wirksam bei der Hemmung der Korrosion von in Berührung mit Frostschutz- oder wässrigen Hydraulikflüssigkeitszusammensetzungen stehenden Metallen.

Bei den wässrigen, erfindungsgemässen Funktionsflüssigkeitsformulierungen kann die erfindungsgemässe Korrosionsschutzmittelzusammensetzung für sich allein oder als Gemisch mit anderen funktionellen Zusätzen, z.B. weiteren Korrosionsschutzmitteln und/oder Höchstdruckzusätzen eingesetzt werden.

Weitere in Betracht kommende Korrosionsschutzmittel

gehören beispielsweise zu folgenden Gruppen:
a) Organische Säuren, deren Ester oder Ammonium-, Amin-, Alkanolamin- und Metallsalze, zum Beispiel Benzoesäure, p-tert.-Butylbenzoesäure, Dinatriumsebacinat, Triäthanolaminlaurat, Isononansäure, das Triäthanolaminsalz der p-Toluolsulfonamid-capronsäure, Natrium-N-lauroylsarkosinat oder Nonylphenoxyessigsäure, sowie Natriumborat, Natriumnitrat, Natriumnitrit und Natriummetasilikat;
b) stickstoffhaltige Materialien wie solche der folgenden Typen: Fettsäurealkanolamide, Imidazoline, z.B. 1-Hydroxyäthyl-2-oleyl-imidazolin, Oxazoline, Triazole, z.B. Benztriazol und Tolutriazol, und Fettamine;
c) phosphorhaltige Materialien wie solche der folgenden Typen: Aminphosphate, Phosphonsäuren oder anorganische Salze, beispielsweise Natriumdihydrogenphosphat, und
d) schwefelhaltige Verbindungen wie solche der folgenden Typen: Natrium-, Calcium- oder Bariumpetroleumsulfonate oder heterocyclische Verbindungen, beispielsweise Natriummerkaptobenzthiazol.

Beispiele für gegebenenfalls vorhandene Höchstdruckzusätze sind unter anderem Schwefel und/oder Phosphor und/oder Halogen enthaltende Materialien, zum Beispiel sulfuriertes Spermöl, sulfurierte Fette, Tritolylphosphat, chlorierte Paraffine oder äthoxylierte Phosphatester.

Die nachfolgenden Beispiele dienen der näheren Erläuterung der vorliegenden Erfindung. Teile und Prozentangaben sind dabei Gewichtsteile und Gewichtsprozente; Drucke sind in Millibar angegeben.

Beispiel 1

A) 3-(1,1,3,3-Tetramethylbutyl)-adipinsäure wird nach der Methode von Niederl und Smith, J. Am. Chem. Soc. 59, 715-7, (1937) hergestellt und weist einen Schmelzpunkt von 136-9°C auf (Literatur 133-4°C).

B) Triäthanolamin und Wasser werden dem Produkt aus Teil A) in Gewichtsverhältnisanteilen von Produkt aus Teil A: Triäthanolamin:Wasser wie 5:5:3 zugesetzt.

Beispiel 2

A) 22 Teile handelsübliches 4-Nonylphenol, 1,0 Teil Platinoxid und 200 Teile Eisessig werden bei Raumtemperatur und Atmosphärendruck 24 Stunden lang mit Wasserstoff geschüttelt. Nach Ablauf dieser Zeit hat die Wasserstoffaufnahme aufgehört, und die Lösung wird vom Katalysator abfiltriert und die Essigsäure bei vermindertem Druck entfernt. Das verbleibende Oel wird dann destilliert, was 6,2 Teile einer Fraktion mit Siedepunkt bis 160°C/12 Torr und danach 12,9 Teile gemischte 4-Nonylcyclohexanolisomere mit Siedepunkt 163-7°C/12 Torr mit der folgenden Gewichtsprozentzusammensetzung liefert:

| | Kohlenstoff | Wasserstoff |
|---|---|---|
| Gefunden | 79,66 | 13,97 |
| Berechnet für $C_{15}H_{30}O$ | 79,58 | 13,36 |

Man tropft 10 Teile 4-Nonylcyclohexanol unter Rühren im Verlauf von 1,5 Stunden zu 120 Teilen 0,1 Teil Ammoniummetavanadat enthaltender 70%iger Salpetersäure, wobei man die Temperatur auf 75°C hält. Nach beendeter Zugabe rührt man noch 2 Stunden bei 75°C weiter, bevor man das Reaktionsgemisch in 1 Liter Wasser giesst. Das abgeschiedene Oel wird mit Aether extrahiert, mit Wasser gewaschen und eingedampft, wobei 12,1 Teile 3-Nonyladipinsäureisomerengemisch mit der folgenden Zusammensetzung in Gewichtsprozent verbleiben.

| | Kohlenstoff | Wasserstoff |
|---|---|---|
| Gefunden | 64,17 | 10,20 |
| Berechnet für $C_{15}H_{28}O_4$ | 66,14 | 10,36 |

B) Dann setzt man in der in Beispiel 1 (A) beschriebenen Weise Triäthanolamin und Wasser dazu.

Beispiel 3

A) Bei 80°C leitet man unter Rühren einen gleichmässigen Strom Bortrifluorid 4½ Stunden lang in ein Gemisch aus 94 Teilen Phenol und 72 Teilen n-Octansäure. Nach dieser Zeit giesst man das Reaktionsgemisch in Wasser und extrahiert die organische Phase mit Aether. Die

ätherische Lösung wird dann mit Wasser, Natriumbicarbonatlösung und Wasser gewaschen und eingedampft. Destillation des Rückstands gibt als Vorlauf 43 Teile einer Fraktion mit Siedepunkt bis 160°C/12 Torr, die hauptsächlich aus zurückgewonnenem Phenol besteht, und danach 104 Teile einer Fraktion mit Siedepunkt 166-84°C/0,2 Torr, die ein Gemisch aus 2- und 4-n-Octanoylphenol darstellt. Behandlung dieser Endfraktion mit etwas Aether enthaltendem Petroläther (Siedepunkt 40-60°C) ergibt 80 Teile 4-n-Octanoylphenol mit Schmelzpunkt 61-4°C und der folgenden Zusammensetzung in Gewichtsprozent.

| | Kohlenstoff | Wasserstoff |
|---|---|---|
| Gefunden | 76,60 | 9,22 |
| Berechnet für $C_{14}H_{20}O_2$ | 76,33 | 9,15 |

Man beschickt einen Autoklaven mit 50 Teilen 4-n-Octanoylphenol, 5 Teilen 5% Ru/C und 160 Teilen Methanol. Nach Spülung mit Stickstoff wird der Autoklav unter 80 Atmosphären Wasserstoffdruck gesetzt und 44 Stunden bei 125°C gerührt. Nach Abkühlung wird der Autoklav entleert, der Katalysator von der Methanollösung abfiltriert und das Methanol am Rotationsverdampfer bei vermindertem Druck entfernt. Das zurückbleibende Oel wird destilliert und gibt als Vorlauf 3,3 Teile mit Siedepunkt bis 148°C/12 Torr und danach 35,6 Teile 4-n-Octylcyclohexanol mit Siedepunkt 150-4°C/12 Torr und der folgenden Zusammensetzung in Gewichtsprozent.

| | Kohlenstoff | Wasserstoff |
|---|---|---|
| Gefunden | 79,11 | 12,99 |
| Berechnet für $C_{14}H_{28}O$ | 79,18 | 13,29 |

Bei 70-5°C tropft man unter Rühren im Verlauf 1 Stunde 44 Teile 4-n-Octylcyclohexanol zu einer Lösung von 500 Teilen 70%iger (Gew./Gew.) Salpetersäure und 0,1 Teil Ammoniummetavanadat. Nach beendeter Zugabe wird noch 1 Stunde bei 70-5°C weitergerührt, bevor man das abgekühlte Reaktionsgemisch in 1 000 Teile Wasser giesst. Das abgeschiedene Oel erstarrt und wird filtriert, gewaschen und

getrocknet, was 49,7 Teile unreine 3-n-Octyladipinsäure vom Schmelzpunkt 65-9°C ergibt. Durch Kristallisieren der Säure aus etwas Aether enthaltendem Petroläther (Siedepunkt 40-60°C) erhält man 35,1 Teile reine Säure mit Schmelzpunkt 72-4°C und der folgenden Zusammensetzung in Gewichtsprozent.

| | Kohlenstoff | Wasserstoff |
|---|---|---|
| Gefunden | 65,01 | 10,32 |
| Berechnet für $C_{14}H_{26}O_4$ | 65,09 | 10,14 |

B) Dann setzt man in der in Beispiel 1 (A) beschriebenen Weise Triäthanolamin und Wasser dazu.

Beispiele 4-6

Die Korrosionsbeständigkeit einer erfindungsgemässen wässrigen Schneidflüssigkeitszusammensetzung wird nach der folgenden Arbeitsweise beurteilt, welche eine Modifikation des Tests 287 des Institute of Petroleum darstellt. Man stellt eine 1%ige wässrige Lösung des zu prüfenden Korrosionsschutzmittels her, die genügend Triäthanolamin (TEA) enthält, um ihren pH-Wert auf 9 zu bringen.

Diese Lösung wird mit entionisiertem Wasser um Faktoren 2, 4, 8 und 16 weiter verdünnt, und diese Lösungen werden jeweils nach der in der Testvorschrift IP 287 angegebenen Methode mit auf Filterpapier gelegten Gusseisenspänen in Berührung gebracht. Nach 2 Stunden entfernt man die Gusseisenspäne und untersucht das Filterpapier auf gelbe oder grüne Flecken, welche auf Rosten des Metalls hinweisen würden. Die erhaltenen Ergebnisse sind in Tabelle 1 angeführt.

Tabelle 1 Hemmung der Korrosion von Gusseisen
Wässrige Schneidflüssigkeiten

| Beispiel | Verbindung | % TEA auf 1% der Verbindung | pH | IP-287-Test | | |
|---|---|---|---|---|---|---|
| | | | | Verdünnung* | | Rost + (entionisiertes Wasser) |
| | | | | Verhältnis | % | |
| 4 | 3-(1,1,3,3-Tetramethylbutyl)-adipinsäure | 6,7 | 9,0 | 1:104<br>1:208 | 0,125<br>0,062 | 0<br>0 |
| 5 | 3-n-Octyladipinsäure | 6,7 | 9,0 | 1: 52<br>1:104<br>1:208 | 0,25<br>0,125<br>0,062 | 0<br>0<br>0 |
| 6 | 3-Nonyladipinsäure | 6,7 | 9,0 | 1: 52<br>1:104<br>1:208 | 0,25<br>0,125<br>0,062 | 0<br>0<br>0 |

* Das Verdünnungsverhältnis ist das Verhältnis von Triäthanolamin + Verbindung zu Wasser

% bedeutet die % Verbindung in der untersuchten Lösung

+ 0 bedeutet kein nachweisbarer Rost

Beispiel 7

Typische Metallproben für solche, die im Kühlsystem eines Verbrennungsmotors vorhanden sein könnten, werden zwei Wochen bei 82°C völlig in eine belüftete Testlösung eingetaucht. Die Korrosionsschutzeigenschaften der Lösung werden aufgrund der von Metallproben erlittenen Gewichtsverluste ausgewertet.

Der verwendete Apparat ist in der beigefügten Figur 1 abgebildet und wie folgt aufgebaut:

1 000 ml-"Kulturgefäss",
Flanschdeckel mit mehreren Stutzen,
auf den Flanschdeckel passender, wassergekühlter Rückflusskühler,
Gasverteilungsrohr mit Frittenkopf vom Porositätsgrad Nr. 2, auf einen Stutzen des Flanschdeckels passend,
zur Regulierung der Belüftungsgeschwindigkeit auf 100 ± 15 ml pro Minute geeigneter Strömungsmesser,
auf einen Stutzen des Flanschdeckels passendes Thermometer für den Bereich -5 bis 105°C,
Druckluftflasche,
Wasserbad zur Einhaltung einer Temperatur von 82° ± 2°C und
auf das Wasserbad passende Metallplatte, so dass man 6 Kulturgefässe unterbringen kann.

Die Metallproben (50 mm x 25 mm mit 6 mm Loch in der Mitte) sind wie folgt:

i) Stahl kaltgewalztes Stahlmaterial U.S. SAE 1 020
ii) Kupfer Typ ETP oder STP nach U.S. Norm für Kupferblech, -platten oder -walzstab nach ASTM B 152, kaltgewalztes Stahlmaterial SAE 71.
iii) Messing
Legierung Nr. 8 der U.S. Norm für Messingplatten, -blech, -band und -walzstab, ASTM B 36, SAE 70C.
iv) Lötmetall
Legierungsqualität 30A oder 30B nach U.S. ASTM B32

v) Aluminiumguss

Legierung SC 64C nach U.S. ASTM B 179, SAE 329

vi) Gusseisen

Legierung Nr. 120 nach U.S. ASTM 159 SAE 120.

Sämtliche Metallproben werden gründlich mit Bimsstein und einer feuchten Borstenbürste gescheuert, mit kaltem Wasser und dann Aceton abgespült, getrocknet und gewogen.

Die Proben werden auf einer Maschinenschraube aus Messing angeordnet, die mit dünnwandigem Isolierschlauch so kleinen Durchmessers bedeckt ist, dass sie leicht durch das Mittelloch in den Proben gleitet. Diese sind voneinander durch 5 mm lange zylindrische Abstandsstücke mit 11 mm Aussendurchmesser und 6 mm Innendurchmesser getrennt. An den Enden der Anordnung befindet sich jeweils ein 50 mm mal 25 mm grosser, aus 1,5 mm-Messingblech geschnittener Messing-"Arm" mit einem Loch von 6 mm Durchmesser, das exzentrisch gegen ein Ende des Messingstücks liegt und mit seiner Längsachse in der gleichen Richtung wie die Längsachse der Probe orientiert ist (siehe Figur 1).

Die Proben und Arme werden in folgender Reihenfolge (siehe Figur 1) angeordnet: Messing-"Arm", Kupfer, Lötmetall, Messing, Stahl, Gusseisen, Aluminiumguss, Messing-"Arm".

Die Abstandsstücke zwischen den Messing-"Armen" und den benachbarten Proben sowie zwischen den Messing- und Stahlproben bestehen aus isolierendem Material, die zwischen den Messing-, Lötmetall- und Kupferproben aus Messing und die zwischen den Stahl-, Aluminium- und Eisenproben aus Stahl.

Die Anordnung aus "Armen", Abstandsstücken und Proben wird mittels einer Messingmutter so zusammengeschraubt, dass guter elektrischer Kontakt der drei Proben miteinander in jedem Abschnitt sichergestellt ist.

Ein Gewichtsteil der zu prüfenden Frostschutzzusammensetzung wird mit 2 Teilen Wasser verdünnt, das 164 g/l NaCl und 444 mg/l $Na_2SO_4$ enthält.

Das verdünnte Frostschutzmittel (750 ml) gibt man wie in Figur 1 gezeigt in das Gefäss. Dieses wird in ein Wasserbad bei 82° ± 2°C eingetaucht und die Belüftungsgeschwindigkeit auf 100 ± 15 ml pro Minute eingestellt.

Die Prüfung wird 336 Stunden lang (2 Wochen) fortgesetzt. Nach Beendigung der Prüfung wird die Probenanordnung entfernt und auseinandergenommen. Die einzelnen Metallproben werden unter Abbürsten zwecks Entfernung lose gehaltener Korrosionsprodukte in fliessendem Wasser gewaschen. Fester anhaftende Produkte werden wie folgt entfernt:

Eisen und Stahl

Die Probe wird 1 Minute in 1% Hexamin enthaltende 50%ige HCl-Lösung getaucht.

Kupfer und Messing

Die Probe wird 30 Sekunden in 30%ige HCl-Lösung eingetaucht.

Aluminiumlegierung

Die Probe wird 5 Minuten in eine bei 80°C gehaltene Lösung getaucht, die 2% Chromsäure und 5% $H_3PO_4$ enthält.

Lötmetall

Die Probe wird 5 Minuten in kochende 1%ige Essigsäurelösung eingetaucht.

Die Schlussreinigung sämtlicher Proben wird durch Scheuern mit einer feuchten Bürste durchgeführt. Nach gründlicher Reinigung werden die Proben in Aceton gespült, getrocknet und gewogen.

Es wird angenommen, dass die Proben diese Prüfung bestanden haben, wenn der Korrosionsverlust kleiner als die folgenden Zahlen ist:

| | |
|---|---|
| Kupfer | 0,31 $mg/cm^2$ |
| Lötmetall | 0,62 $mg/cm^2$ |
| Messing | 0,31 $mg/cm^2$ |
| Stahl | 0,31 $mg/cm^2$ |
| Gusseisen | 0,31 $mg/cm^2$ |
| Al-Legierung | 1,08 $mg/cm^2$ |

Eine wie folgt hergestellte Frostschutzzusammensetzung:

| | | |
|---|---|---|
| Dikaliumsalz aus Beispiel 1A | 4 | Gewichtsteile |
| Benztriazol | 0,2 | Gewichtsteile |
| Aethylenglykol | 95,8 | Gewichtsteile |

gibt bei der Prüfung nach der oben beschriebenen Methode die folgenden Ergebnisse.

| Metall | Gewichtsverlust mg/cm² |
|---|---|
| Kupfer | <0,007 |
| Lötmetall | 0,17 |
| Messing | 0,008 |
| Stahl | 0,02 |
| Gusseisen | 0,03 |
| Al—Legierung | 0,10 |

Beispiel 8

Die Wirksamkeit eines Rostschutzmittels in einer Hydraulikflüssigkeit auf Wassergrundlage lässt sich unter Anwendung der Apparatur und einer Modifikation der Methode nach ASTM D665 zur Bestimmung der "Rust-preventing Characteristics of a Steam turbine oil in the presence of Water [Rostverhindernde Kenndaten eines Dampfturbinenöls in Gegenwart von Wasser]" bestimmen.

Bei dieser Modifikation wird eine Normstahltestspindel gereinigt und 24 Stunden in ein Becherglas gehängt, welches die gerührte und auf 60° erhitzte, zu prüfende Flüssigkeit enthält. Nach Beendigung der Prüfung wird die Spindel auf Rostanzeichen untersucht.

Es werden die folgenden Flüssigkeiten hergestellt:

| | A | B | C |
|---|---|---|---|
| Entionisiertes Wasser | 40 Gew.T. | 40 Gew.T. | 40 Gew.T. |
| Aethylenglykol | 40 " " | 40 " " | 40 " " |
| UCON PEG 75H-90000 | 20 " " | 20 " " | 20 " " |
| Dinatriumsalz aus Beispiel 1A | - | 0,1 " " | - |
| Produkt aus Beispiel 1B | - | - | 0,1 " " |

und bei der Prüfung nach der oben beschriebenen Methode erhält man die folgenden Ergebnisse.

| Flüssigkeit | Aussehen der Testspindel |
| --- | --- |
| A | 6 tiefe Rostflecken sowie 30% der Oberfläche milchig verfärbt |
| B | Oberfläche sauber und glänzend |
| C | Oberfläche sauber und glänzend |

Patentansprüche:

1. Korrosionsschutzmittelzusammensetzung zur Hemmung der Korrosion von Metallen, gekennzeichnet durch

(a) mindestens eine 3-Alkyladipinsäure der Formel

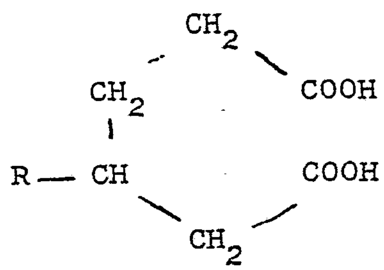

I

worin R eine geradkettige oder verzweigte $C_4$-$C_9$-Alkylgruppe bedeutet,

(b) ein Basifizierungsmittel und gegebenenfalls

(c) Wasser,

wobei das Gewichtsverhältnis der Verbindung der Formel I zum Basifizierungsmittel im Bereich 1:1 bis 1:20 liegt.

2. Zusammensetzung nach Anspruch 1, dadurch gekennzeichnet, dass als Verbindung der Formel I 3-n-Butyladipinsäure, 3-t-Butyladipinsäure, 3-n-Pentyladipinsäure, 3-(1,1-Dimethylpropyl)-adipinsäure, 3-n-Hexyladipinsäure, 3-n-Heptyladipinsäure, 3-n-Octyladipinsäure, 3-(1,1,3,3-Tetramethylbutyl)-adipinsäure oder 3-Nonyladipinsäure vorliegt.

3. Zusammensetzung nach Anspruch 1, dadurch gekennzeichnet, dass R eine geradkettige oder verzweigte $C_8$-$C_9$-Alkylgruppe bedeutet.

4. Zusammensetzung nach einem der vorhergehenden Ansprüche, dadurch gekennzeichnet, dass als Basifizierungsmittel eine wässrige Lösung eines Alkalimetallhydroxyds vorliegt.

5. Zusammensetzung nach einem der Ansprüche 1 bis 3, dadurch gekennzeichnet, dass als Basifizierungsmittel Triäthanolamin vorliegt.

6. Funktionsflüssigkeitsformulierung, dadurch gekennzeichnet, dass sie als Korrosionsschutzmittel eine Korrosionsschutzmittelzusammensetzung nach Anspruch 1 enthält.

7. Formulierung nach Anspruch 6, dadurch gekennzeichnet, dass sie eine wie hierin definierte Metallbearbeitungsformulierung ist.

8. Formulierung nach Anspruch 6, dadurch gekennzeichnet, dass sie eine Frostschutzformulierung ist.

9. Formulierung nach Anspruch 6, dadurch gekennzeichnet, dass sie eine Hydraulikflüssigkeitsformulierung auf Wassergrundlage ist.

10. Formulierung nach einem der Ansprüche 6 bis 9, dadurch gekennzeichnet, dass die Korrosionsschutzmittelzusammensetzung gemäss Anspruch 1 für sich allein oder als Gemisch mit anderen funktionellen Zusätzen eingesetzt wird.

11. Formulierung nach Anspruch 10, dadurch gekennzeichnet, dass die funktionellen Zusätze weitere Korrosionsschutzmittel und/oder Höchstdruckzusätze sind.

12. Verbindungen der Formel

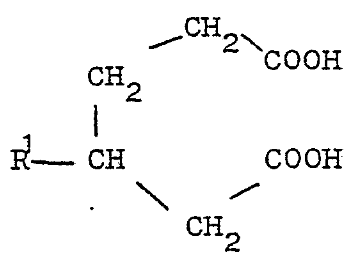

II

worin R' geradkettiges oder verzweigtes $C_7$-$C_9$-Alkyl bedeutet, jedoch mit Ausnahme von t-Octyl.

0060224

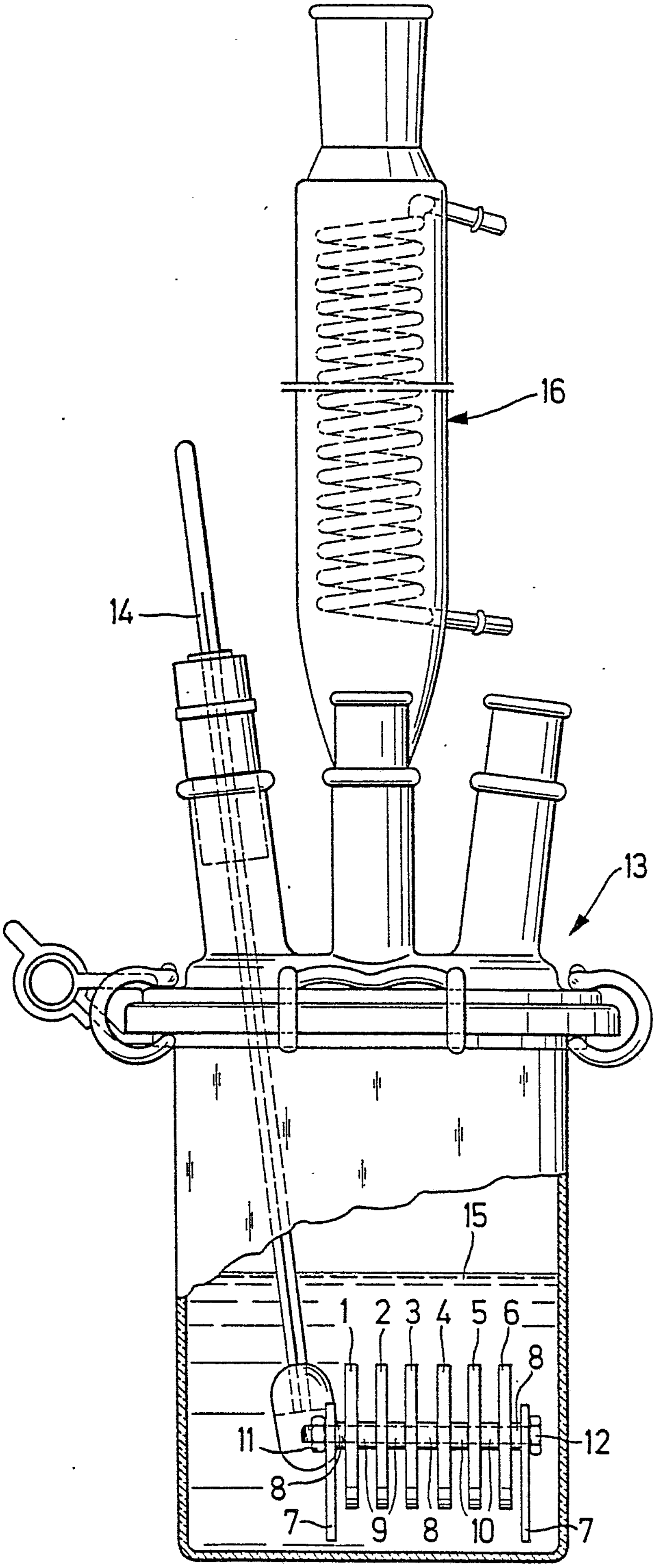

0060224

Nummer der Anmeldung

Europäisches Patentamt

# EUROPÄISCHER RECHERCHENBERICHT

EP 82810101.4

| EINSCHLÄGIGE DOKUMENTE | | | KLASSIFIKATION DER ANMELDUNG (Int Cl.³) |
|---|---|---|---|
| Kategorie | Kennzeichnung des Dokuments mit Angabe, soweit erforderlich, der maßgeblichen Teile | betrifft Anspruch | |
| P,X | EP - A1 - 0 029 892 (CHEMISCHE WERKE HULS AG)<br>* Gesamt; insbesondere Seiten 2,4; Patentansprüche *<br>-- | 1-12 | C 23 F 11/12<br>C 23 F 11/14<br>C 09 K 5/00<br>C 07 C 55/02<br>C 10 M 3/18 |
| Y | US - A - 2 426 496 (F.F. FARLEY)<br>* Spalte 2; Patentansprüche *<br>-- | 1,2 | |
| Y | US - A - 3 981 780 (C-J. SCHERRER)<br>* Spalte 2, Zeilen 30-38; Patentansprüche, insbesondere 4 *<br>---- | 1,2 | RECHERCHIERTE SACHGEBIETE (Int. Cl.³)<br>C 23 C<br>C 09 K<br>C 07 C<br>C 10 M |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| WIEN | 01-06-1982 | SLAMA |